# EUROPEAN PATENT SPECIFICATION

(11) **EP 2 446 262 B1**
(45) Date of publication and mention of the grant of the patent: **10.09.2014**
(21) Application number: 10731805.7
(22) Date of filing: 23.06.2010
(51) Int. Cl.: G01N 33/483

(54) **PROCESSES AND DEVICES FOR DETERMINING BODY PARAMETERS**
VERFAHREN UND VORRICHTUNGEN ZUR BESTIMMUNG VON KÖRPERWERTEN
MÉTHODES ET DISPOSITIFS UTILISÉS POUR DÉTERMINER DES PARAMÈTRES BIOLOGIQUES

(30) Priority: 27.06.2009 DE 102009030765
(43) Date of publication of application: 02.05.2012
(73) Proprietor: Braun GmbH, 61476 Kronberg/Taunus (DE)
(72) Inventor: CEPUS, Valentin, 61479 Glashuetten (DE); STEGHAUS, Michael, 60437 Frankfurt (DE)
(74) Representative: Sievers, Uwe
(86) International application number: PCT/IB2010/052865
(87) International publication number: WO 2010/150210

(56) References cited:
- EP-A1- 1 839 573
- WO-A2-2005/053514
- AU-B2- 633 952
- DE-C1- 4 402 237
- JP-A- 9 327 269
- US-A1- 2005 258 285

## Description

The invention relates to processes and devices for determining body parameters in which body hair removed by shaving is used, in particular facial hair.

In the prior art it is quite generally known that because of the composition of body hair, inferences about body parameters can be drawn from it. For human hair growth, nutritional substances are drawn from the blood and accumulate in the hair follicles. Along with the nutritional substances, minerals and trace elements also enter the growing hair shaft from the blood. In the hair, concentrations of some trace elements occur up to ten fold greater than in the blood.

Moreover, various processes for analysis of trace elements are generally known. Trace elements can be analyzed spectrophotometrically, among other ways. A well known process is spectro-analysis using an emission spectrometer that works with inductively coupled plasma. The plasma is usually a chemically inert gas, wherein argon lends itself well because of its high potential for ionization, its relatively low cost and its lack of band spectra. Samples to be analyzed are sprayed into the plasma with a sample atomizer and excited by the plasma. These samples emit characteristic spectral lines that can be measured with a spectrometer.

Furthermore, various shaving systems are very well known. Besides wet shaving, dry or electric shaving enjoys great popularity. This kind of shaver usually works with an electric drive and a shaver or shaving system powered by the electric drive. Typically, a combination of upper and lower blades is used, wherein the lower blade moves in opposition to the upper blade. Shavers that can be used wet also belong to this class of shaver, nevertheless they can be assigned to the above description of the dry or electric shavers due to their manner of operation.

In addition, a cleaning device for cleaning the shaving head of a dry shaver is known from DE 44 02 237 C1, in which a shaving head of the electric shaver is inserted into a cleaning station and is flushed with a cleaning fluid by a motor-driven delivery device. The accumulated hair residue in the shaving head of the electric shaver is filtered out of the cleaning fluid through a filtering device in the cleaning station.

See also for example JP 09 - 327269, WO 2005/053 514 A2, AU-B-60970/90, EP 1839 573 A1 and US 2005/0258 285 A1.

There is a desire to integrate the monitoring of bodily functions into everyday life so that when values deviate from the norm, a quick diagnostic tool is ready to permit a modification of the health condition of a patient.

The object is to provide processes and devices of the type mentioned above, that are easy to integrate into everyday life and that offer the user a way to monitor body parameters without imposing an additional burden on the user.

The invention is achieved by means of a process for determining body parameters according to Claim 1, a device for determining body parameters according to coordinate claim 8 and a use of a shaving system according to coordinate claim 13. Advantageous embodiments are the subject matter of the dependent claims.

A process for determining body parameters according to the invention is based on the use of body hair removed by a shaver. Additional body substances are removed along with the body hair removed by the shaver, for example, skin particles, fat and/or perspiration. The body substances adhere to the hair or are picked up by the razor during the process of shaving.

In the context of the invention, body parameters are primarily the presence and concentration of specific elements and compounds as well as other chemical or physical quantities such as pH value, for example, or other similar quantities.

According to the invention, the body hair, in particular facial hair, is separated from the other body substances adhering thereto or which are removed by the shaver, so that the body hair on the one hand and the body substances on the other hand can be analyzed separately.

The isolated or separate body hair is evaluated for its mineral content, while the removed body substances are analyzed separately for their pH value and/or for their conductance.

Today it is possible to measure calcium, magnesium, potassium, copper, zinc, iron, chromium, iodine, selenium, cobalt, molybdenum, vanadium, lead, mercury, cadmium, arsenic, nickel, manganese, silicon, barium, phosphorus, tin, bismuth, strontium, sodium, boron, lithium, silver, and thallium, for example.

Deviations in the concentrations of these elements or of some of these elements suggest inferences about deviations in bodily functions. For example, a reduced level of calcium in the hair may indicate declining bone density. Too little iron can be an indicator of anemia. Elevated levels of mercury, cadmium, arsenic, bismuth, strontium, thallium, in turn can be warning signs of poisoning.

Moreover, in addition or alternatively, the pH value of the isolated or separated body substances collected during shaving is analyzed. The pH provides evidence concerning the condition of the skin and inferences can possibly be drawn about the acid balance in the body. In this way, both health monitoring can be undertaken, and the fitness level of the user can measured, because a poor fitness level is often accompanied by body acidification.

In addition or alternatively, the conductance and the temperature of the solution are measured. With the aid of a conductivity measurement, other body parameters are determined, for example, an acetate-lactate ratio and a metal ion concentration can be measured, from which a global parameter value of minerals can be determined. In this way, for example, low mineral concentrations due to poor nutrition can be determined.

The results of the process can be used to inform the user about the body parameter, so that, when changes occur in his body parameters, he receives early information about the state of his body. By knowing about deviations in the parameters, one can consult a doctor early on for a diagnosis. Furthermore, users can monitor their fitness levels, for example, as well as changes in their fitness level over time. Moreover, statistical analysis of changes in body parameters can be carried out. Finally, it is conceivable that when certain parameters deteriorate, information will be given about opportunities for future improvement of the parameters.

According to a first advantageous embodiment of the invention, body hair is removed along with the body substances removed by a shaver, in particular a dry or electric shaver, wherein the analysis of the body parameters are carried out in a cleaning device of the shaver. In an advantageous embodiment, a cleaning device, which can be configured as a cleaning station, can be provided with the required devices and components for analysis of the body parameters. In addition, it is easier to supply a cleaning device, particularly a cleaning station, with the energy required for analysis.

Another advantage of using a cleaning device or cleaning station is that the purpose of such cleaning devices is precisely to free the razor of body hair and body substances. These are thus already incorporated into the cleaning device.

In this way, the next time the procedure is performed, it can be ensured that no hair and body substances remain from the last use of the razor, which could skew results.

Alternatively, body parameters can be determined in the shaver itself.

Preferably, body hair and body substances are separated in a liquid, in particular in acetone.

Preferably, the length of the removed body hair is measured. Using the measurement of the length of the removed body hair can indicate the period of time to which the body parameters relate, since the rate of hair growth is relatively constant. In addition, it may be preferred to calibrate the system by correlating the measured hair length with the time intervals between successive cleaning cycles.

For separating body hair and body substances from each other, suspending the hair and body substances removed by shaving in a solution and then filtering is particularly preferred. By means of filtration, it is particularly easy to separate hair from the other body substances, which tend to be composed of much smaller particles.

Alternatively, the difference in hydrodynamic volumes of hair and of body substances can be used to separate these from the hair in a slight counter current of an acetone solution, for example.

Another possibility is to subject the suspended body hair and body substances to ultrasonic waves one or more times, wherein the body substances aggregate in the supernatant. The hair particles, in contrast, drop out.

The above processes for separation can be achieved very easily in small scale devices.

A preferred process for analysis of body hair is the use of spectroscopic examination processes. What is known as the ICP-OES process (inductive coupled plasma, optical emission spectroscopy) has proven to be particularly suitable for this purpose: In this process, body hair suspended in a solution is sprayed into plasma, in particular argon plasma, and the spectral lines of the suspended body hair in the solution excited by the plasma are measured, preferably with respect to frequency and amplitude. In this way, the presence and concentration of certain substances, such as minerals, are detected.

Another possible embodiment of a spectrometer is a UV-VIS spectrometer, by means of which a weakening of a hair solution can be determined. From this weakening, conclusions can be drawn about the mineral content of the solution, whereby a global parameter can be determined.

To dissolve the body hair, it is preferably decomposed with nitric acid and then reduced to ashes by microwave. This process ensures complete disintegration of the body hair and denaturation of all organic components.

A clear, colorless solution is obtained by means of the described process of disintegration and reduction to ash, in which the minerals are present as nitrates. Such a solution is particularly suitable for spectrophotometric analysis.

In addition, fats can advantageously be separated from the body substances and amino acids and/or cholesterol can be measured in the fats. This allows other health-related body parameters to be captured, such as cholesterol content.

Using an advantageous analysis of amino acids, a ratio of hair quantity to skin quantity can be determined. From variations in the concentration of amino acids, conclusions about the health of skin can be drawn. For example, the amino acid concentration can increase with dry or sunburned skin, because skin cells are more easily released by the razor during shaving.

Evidence of a possible amino acid deficiency in the user is revealed by measuring the amino acids; this can be offset by appropriate diet.

A first independent subject matter of the invention relates to an apparatus for determining body parameters with an isolation device for separating body hair shaved with a razor, especially facial hair, from body substances adhering thereto, or body substances removed by the razor, such as skin particles, fat and/or perspiration, and additionally a mineral analysis device to determine the mineral content of body hair and/or a pH sensor to determine the pH of the body substances removed with the hair and/or a device for measuring conductance. Shaving unavoidably creates a material mixture of both body hair and body substances adhering to it or body substances removed by shaving, wherein, if hair components and body substances are separated, further conclusions concerning body parameters can be drawn. Mineral analysis of body hair in particular can be carried out, but it can also establish certain proteins. Other body substances render other conclusions possible, such as those concerning the acid mantle of the skin.

The device according to the invention preferably conveys the hair separated from the body substances to the hair mineral analysis device which determines the type and concentration of the minerals accumulated in the hair. The separated body substances are delivered to the pH sensor, which provides a pH value thereof. According to a first advantageous embodiment of the invention, a device for measuring hair length is provided so that the length of the severed hair can be measured. The period of time to which the analysis relates can be determined from the length of the hair, taking into account the growth rate. Preferred embodiments of a hair- measuring device are optical, gravimetric or screening devices for measuring hair-length.

A conductance measuring cell preferably works with alternating current, whereby polarization effects can be prevented at the electrodes of the conductance measuring cell. By specifying the temperature for the measured solution, information on conductance can also be improved because conductance variations due to temperature can be taken into consideration.

Preferred embodiments of the separating device are a filtration device and/or a counter-current separation device and/or an ultrasonic separation device. With the help of such separation devices, body substances and body hair can be sufficiently isolated from each other.

Furthermore, the mineral-assay device is preferably attached to a microwave incineration apparatus with which a better solution and distribution of body hair can be achieved, thus increasing analytical accuracy.

Furthermore, the mineral analysis device can have a spectroscope, in particular an emission spectrometer with inductively coupled plasma, in particular argon plasma (ICP-OES). Such a spectroscopic analysis is especially reliable and allows the simultaneous analysis of a variety of minerals. Thus, the analysis is very fast. An ICP-OES is expensive, therefore, according to this embodiment, the ICP-OES is preferably executed in a laboratory, to which the separated body hair is sent.

The use of a UV-VIS spectrometer is another possible embodiment of a spectrometer, with which a weakening of a hair solution can be determined. From this weakening, conclusions can be drawn about the mineral content of the solution, whereby a global parameter can be determined.

In a particularly advantageous manner, the device is integrated into a cleaning device for razors. This allows body parameters to be determined using a commercially available razor, especially electric or dry razors. In this way, any additional burden on the user is minimized. The device may further include a display panel on which the user can identify the displayed body parameters or on which additional information can be displayed, in particular, additional information related to the body parameters.

Preferably, further means of storage are provided on which the measured body parameters can be saved. From this, statistics can be derived. for example, or a progression of different body parameters over a longer time period can be traced.

A further independent object of the invention relates to the use of a shaving system having a dry or electric shaver for determining body parameters. Using a razor, the necessary body hair and body substances are very easily collected and subsequently evaluated.

An advantageous development of the use requires that a cleaning station of the dry or electric shaver be used to carry out said body parameters on the body hair and body substances collected from the shaver.

Other objectives, features and advantageous applications of the invention will be apparent from the following description of an embodiment based on the illustrations. All described and/or illustrated features in any reasonable combination form the subject matter of the present invention, even independently of the claims and references.
Fig. 1 shows a flow chart of the device according to the invention according to a first embodiment;
Fig. 2 shows a device according to the invention according to a first embodiment;
Fig. 3 shows a device according to the invention according to a second embodiment;
Fig. 4 shows a device according to the invention according to a third embodiment;

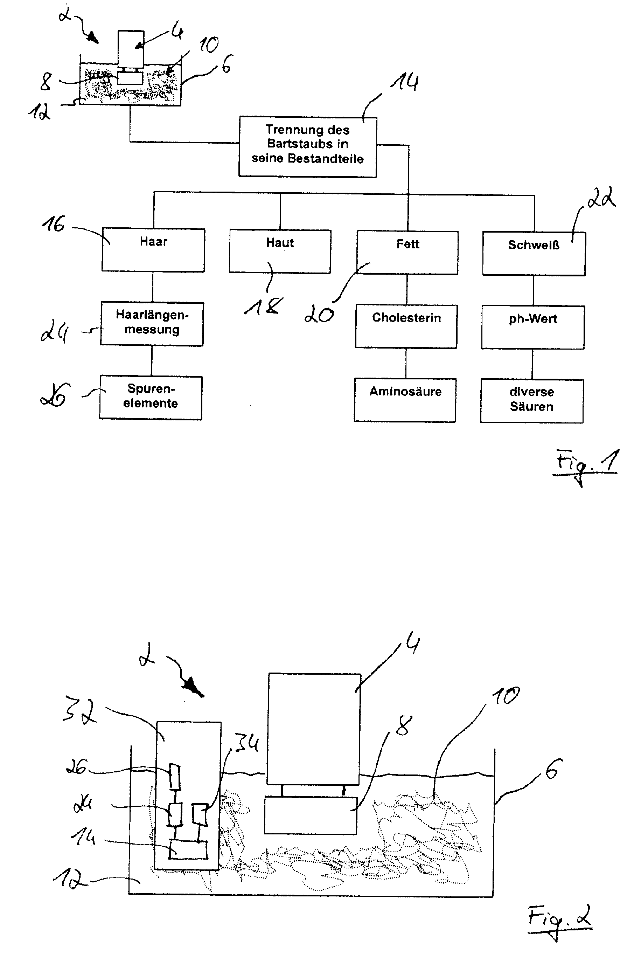

Fig. 1 shows a schematic representation of a flow chart of the process according to the invention for determining body parameters.

In a shaving system 2 with a shaver 4 and a cleaning station 6 an analysis device according to the invention is provided, not shown in Figure 1. A shaving head 8 of the shaver 4 is cleaned in the cleaning station 6 and thereby the shaved body hair 10 is distributed in cleaning solution 12.

With the help of a separation device 14, the body hair 10, mixed with other body substances such as loose skin cells either adhering to the body hair 10 or taken up as the shaving head 8 passed over the skin, are separated from each other.

To achieve separation, body hair 10 and body substances adhering thereto or removed therewith can be suspended and then filtered. It is also possible to make use of the different hydrodynamic volumes and to carry out ultrasound on the facial hair, for example, in a light acetone counter-stream or by repeated treatment of the suspended hair and skin particles, preferably in acetone. As a result, several separate components are obtained, namely hair components 16, skin components 18, and body fats 20, as well as perspiration 22.

The hair can be fed into a hair-length measuring device 24, with the aid of which the period covered by the analysis can be established, because hair growth for each individual is constant. For measuring hair length, optical, gravimetric or a screening device may be used. Furthermore, the hair can be analyzed for trace elements in a trace element analysis system 26. Different measurement methods are used for trace element analysis in the cleaning station 6, for example, or in the LTV-VIS-flow cells integrated into the shaver 4, or more precise and detailed measurement processes in laboratories, for example, those based on spectroscopic studies, such as ICP-OES processes. Such trace element analyses can provide information about deficiencies in the body.

Firstly, the quantity proportion of the removed body hair 10 to skin elements 18 can be determined. From the ratio of body hair to skin particles some conclusions about skin dryness can be drawn, because with increasing skin dryness, the resulting looser skin particles are more easily gathered during shaving.

By analyzing the fat adhering to the hair 16 and to the skin 18 and by isolating the separated fats 20 it is possible to firstly determine cholesterol level and to give feedback concerning the cholesterol level.

In addition, using body fats 20, amino acids can be analyzed, which indicate deficiencies in the body.

The separated perspiration 22 affects the pH of the cleaning solution, which is altered by the perspiration. Using pH values, conclusions can be drawn about the amount of perspiration and thus about a changing fitness level. The perspiration 22 also contains various other acids such as lactic acid, acetic acid and butyric acid.

Fig. 2 shows shaving system 2 according to the invention of Fig. 1 in an enlarged and expanded drawing. An analysis system 32 is integrated into the cleaning station 6, which includes the separation device 14 and the other analytical devices 24 and 26.

In addition, the analysis system 32 is provided with a pH sensor 34, with which the pH of the solution can be ascertained, this being determined largely by the amount and composition of perspiration.

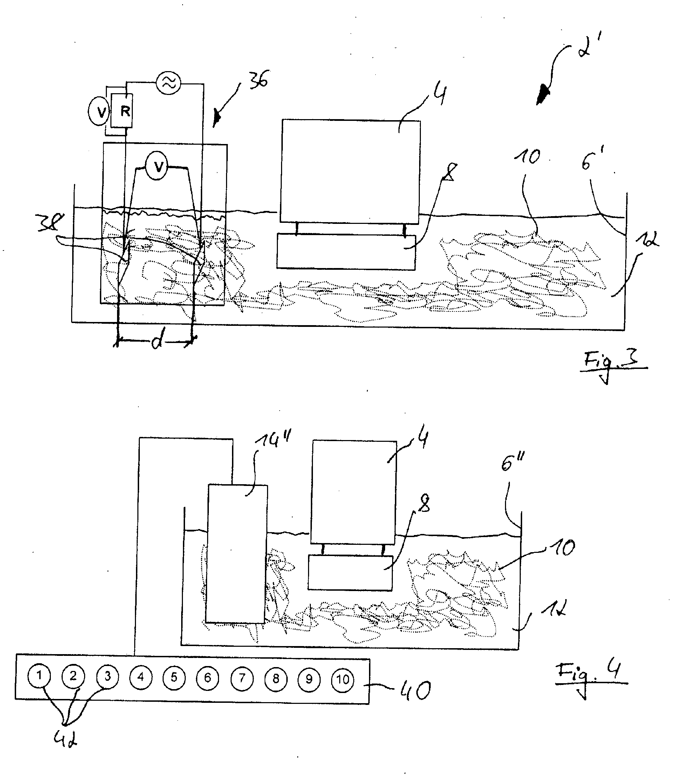

Fig. 3 shows an alternative shaving system 2' with a razor 4 having shaving head 8, which is cleaned in a cleaning station 6'. A conductivity measuring cell 36 is integrated into the cleaning station 6' by means of two electrodes 38 arranged at a predetermined distance d, the conductance of the solution is measured by an AC voltage applied at the electrodes, and current and voltage is measured in the solution 12 mixed with hair 10. In this way, the perspiration secreted by the user of the razor can be determined, as well as the condition of the cleaning liquid. When measuring conductance, it is also necessary to measure the temperature of the solution, which is why a temperature sensor is provided, not shown in Fig 3.

Figure 4 shows a third embodiment of a shaving system 2" according to the invention, in which body hair 10 can be removed from the cleaning solution 12 with the aid of a separating device 14" provided in a cleaning station 6". The body hair 10 separated out can then be collected in a cartridge 40 with several receptacles 42, of which only three of the ten illustrated receptacles are shown with reference numbers.

Each of the compartments 42 is designated for a separate shave, so that in this way ten shaves can be analyzed, for example. The cartridge 40 can be removed from the cleaning station 6" and replaced with a fresh cartridge. The full cartridge 40 can then be delivered to a pharmacy or the like, for example, to be sent to a laboratory for an appropriate analysis.

Further embodiments result from combinations of the illustrated embodiments, in which several of the illustrated measurement principles are combined, for example, a pH measurement with a conductivity measurement.

## Claims

1. A process for determining body parameters, in which body hair removed by shaving, especially facial hair (10), is separated from body substances adhering thereto, or body substances (18, 20, 22) removed separately by shaving, such as skin particles (18), fat (20) and/or perspiration (22), the mineral content of the isolated body hair (10) is measured and/or the isolated body substances (18, 20, 22) removed are analyzed with respect to their pH value and/or their conductance; **characterised in that** the body hair (10) together with the removed body substances (18, 20, 22) are removed by a shaver, especially a dry or electric shaver (4), and the determination of the body parameters is performed in a cleaning device (6, 6 ', 6 ") belonging to the shaver (4).

2. The process of Claim 1, in which body hair (10) and body substances (18, 20, 22) are separated in liquid, preferably in acetone, and the conductance and temperature of the solution are measured.

3. The process according to any of the preceding claims, in which a measurement is made of the length of the removed body hair (10).

4. The process according to any of the preceding claims, in which body hair (10) and body substances (18, 20, 22) are suspended in a solution and filtered in order to separate them from each other or from the body substances (18, 20, 22) are concentrated in the supernatant in a slight counter current or with ultrasonic treatment.

5. The process according to any of the preceding claims, in which the body hair (10) is spectroscopically [measured], in particular by spraying the suspended body hair in a plasma, in particular an argon plasma, and measurement of the excited spectral lines of the suspended body hair, or by UV-VIS measurement.

6. The process according to any of the preceding claims, in which the body hair (10) is dissolved by nitric acid decomposition and reduced to ash by microwaves.

7. The process according to any of the previous claims, **characterized in that** fats (20) are separated from the body substances (18, 20, 22), and amino acids and/or cholesterol are measured.

8. A device for determination of body parameters, with a separation device (14, 14 ") for separating body hair removed by shaving, especially facial hair (10), from body substances (18, 20, 22) adhering thereto, or removed by shaving, such as skin particles, fat and/or perspiration, a mineral analysis device (32) to determine the mineral content of body hair 10) and a pH sensor (34) to determine the pH of body substances (18, 20, 22) removed therewith and/or a conductance measurement device (36)), **characterised in that** the device is integrated into a cleaning station (6, 6 ', 6 ") for a razor (4) or that the device is integrated into a shaver, particularly a dry or electric shaver (4).

9. The device according to Claim 8 with a device for measuring hair-length (24), in particular an optical, gravimetric or screening hair-length measuring device (24).

10. The device according to Claim 8 or 9, wherein the separation device (14, 14") has a filtration device and/or a counter current separation device and/or an ultrasound separation device.

11. The device according to any of Claims 8 through 10, wherein a microwave incineration apparatus is arranged on the mineral analysis device (26).

12. The device according to any Claims 8 through 11, wherein the mineral analysis device (26) has a spectroscope, in particular an emission spectrometer with inductively coupled plasma, in particular argon plasma, or a UV-VIS spectrometer.

13. The use of a shaving system (2, 2 ', 2 ") as defined in any claim 8 to 12, comprising a dry or electric razor, for determining body parameters of its user.

## Patentansprüche

1. Verfahren zum Bestimmen von Körperparametern, wobei durch Rasieren entferntes Körperhaar, insbesondere Gesichtshaar (10), von daran anhaftenden Körpersubstanzen oder Körpersubstanzen (18, 20, 22), die durch das Rasieren separat entfernt wurden, wie Hautteilchen (18), Fett (20) und/oder Schweiß (22), getrennt wird, wobei der Mineralgehalt des isolierten Körperhaars (10) gemessen wird und/oder die isolierten entfernten Körpersubstanzen (18, 20, 22) im Bezug auf ihren pH-Wert und/oder ihre Leitfähigkeit analysiert werden; **dadurch gekennzeichnet, dass** das Körperhaar (10) zusammen mit den entfernten Körpersubstanzen (18, 20, 22) durch einen Rasierer, insbesondere einen Trocken- oder Elektrorasierer (4), entfernt wird und die Bestimmung der Körperparameter in einer Reinigungsvorrichtung (6, 6', 6"), die zu dem Rasierer (4) gehört, erfolgt.

2. Verfahren nach Anspruch 1, wobei Körperhaar (10) und Körpersubstanzen (18, 20, 22) in einer Flüssigkeit, vorzugsweise in Aceton, getrennt werden und die Leitfähigkeit und die Temperatur der Lösung gemessen werden.

3. Verfahren nach einem der vorstehenden Ansprüche, wobei eine Messung der Länge des entfernten Körperhaars (10) durchgeführt wird.

4. Verfahren nach einem der vorstehenden Ansprüche, wobei Körperhaar (10) und Körpersubstanzen (18, 20, 22) in einer Lösung suspendiert und gefiltert werden, um sie voneinander oder von den Körpersubstanzen (18, 20, 22) zu trennen, und in dem Flüssigkeitsüberstand in einem leichten Gegenstrom oder mit Ultraschallbehandlung konzentriert werden.

5. Verfahren nach einem der vorstehenden Ansprüche, wobei das Körperhaar (10) spektroskopisch gemessen wird, insbesondere durch Sprühen des suspendierten Körperhaars in einem Plasma, insbesondere einem Argonplasma, und Messung der angeregten Spektrallinien des suspendierten Körperhaars oder durch UV-VIS-Messung.

6. Verfahren nach einem der vorstehenden Ansprüche, wobei das Körperhaar (10) durch Salpetersäurezersetzung gelöst und durch Mikrowellen zu Asche reduziert wird.

7. Verfahren nach einem der vorstehenden Ansprüche, **dadurch gekennzeichnet, dass** Fette (20) von den Körpersubstanzen (18, 20, 22) getrennt werden und Aminosäuren und/oder Cholesterol gemessen werden.

8. Vorrichtung zur Bestimmung von Körperparametern, mit einer Trennvorrichtung (14, 14") zum Trennen von durch Rasieren entferntem Körperhaar, insbesondere Gesichtshaar (10), von daran anhaftenden oder durch Rasieren entfernten Körpersubstanzen (18, 20, 22), wie Hautteilchen, Fett und/oder Schweiß, einer Mineralanalysevorrichtung (32) zum Bestimmen des Mineralgehalts von Körperhaar 10) und einem pH-Sensor (34) zum Bestimmen des pH-Werts von damit entfernten Körpersubstanzen (18, 20, 22) und/oder einer Leitfähigkeitsmessvorrichtung (36), **dadurch gekennzeichnet, dass** die Vorrichtung in eine Reinigungsstation (6, 6', 6") für einen Rasierer (4) integriert ist oder dass die Vorrichtung in einen Rasierer, insbesondere einen Trocken- oder Elektrorasierer (4), integriert ist.

9. Vorrichtung nach Anspruch 8 mit einer Vorrichtung zum Messen von Haarlänge (24), insbesondere eine optische, gravimetrische oder rasternde Haarlängenmessvorrichtung (24).

10. Vorrichtung nach Anspruch 8 oder 9, wobei die Trennvorrichtung (14, 14") eine Filtrationsvorrichtung und/oder eine Gegenstrom-Trennvorrichtung und/oder eine Ultraschall-Trennvorrichtung aufweist.

11. Vorrichtung nach einem der Ansprüche 8 bis 10, wobei eine Mikrowellen-Einäscherungsvorrichtung auf der Mineralanalysevorrichtung (26) angeordnet ist.

12. Vorrichtung nach einem der Ansprüche 8 bis 11, wobei die Mineralanalysevorrichtung (26) ein Spektroskop, insbesondere ein Emissionsspektrometer mit induktiv gekoppeltem Plasma, insbesondere Argonplasma, oder ein UV-VIS-Spektrometer aufweist.

13. Verwendung eines Rasiersystems (2, 2', 2") nach einem der Ansprüche 8 bis 12, das einen Trocken- oder Elektrorasierer umfasst, zum Bestimmen von Körperparametern seines Benutzers.

## Revendications

1. Procédé pour déterminer les paramètres du corps, dans lequel les poils corporels retirés par rasage, spécialement les poils faciaux (10), sont séparés des substances corporelles adhérentes à ceux-ci, ou des substances corporelles (18, 20, 22) retirées séparément par rasage, telles que des particules de peau (18), de graisse (20) et/ou de perspiration (22), la teneur en minéraux des poils corporels isolés (10) est mesurée et/ou les substances corporelles isolées (18, 20, 22) retirées sont analysées par rapport à leur valeur de pH et/ou leur conductance ; **caractérisé en ce que** les poils corporels (10) conjointement avec les substances corporelles retirées (18, 20, 22) sont retirés avec un rasoir, spécialement un rasoir à sec ou électrique (4), et la détermination des paramètres du corps est effectuée dans un dispositif de nettoyage (6, 6', 6") appartenant au rasoir (4).

2. Procédé selon la revendication 1, dans lequel les poils corporels (10) et les substances corporelles (18, 20, 22) sont séparés dans du liquide, de préférence dans de l'acétone, et la conductance et la température de la solution sont mesurées.

3. Procédé selon l'une quelconque des revendications précédentes, dans lequel une mesure est constituée de la longueur des poils corporels retirés (10).

4. Procédé selon l'une quelconque des revendications précédentes, dans lequel les poils corporels (10) et les substances corporelles (18, 20, 22) sont suspendues dans une solution et filtrées afin de les séparer les unes des autres ou des substances corporelles (18, 20, 22) sont concentrées dans le surnageant dans un léger contre-courant ou avec un traitement ultrasonique.

5. Procédé selon l'une quelconque des revendications précédentes, dans lequel les poils corporels (10) sont de manière spectroscopique [mesurés], en particulier par pulvérisation des poils corporels suspendus dans un plasma, en particulier un plasma d'argon, et la mesure des lignes spectrales excitées des poils corporels suspendus, ou par mesure UV-VIS.

6. Procédé selon l'une quelconque des revendications précédentes, dans lequel les poils corporels (10) sont dissous par décomposition à l'acide nitrique et réduits en cendres par micro-ondes.

7. Procédé selon l'une quelconque des revendications précédentes, **caractérisé en ce que** les graisses (20) sont séparées des substances corporelles (18, 20, 22), et les acides aminés et/ou le cholestérol sont mesurés.

8. Dispositif pour la détermination des paramètres du corps, avec un dispositif de séparation (14, 14 ") pour séparer les poils corporels retirés par rasage, spécialement les poils faciaux (10), des substances corporelles (18, 20, 22) adhérentes à ceux-ci, ou retirés par rasage, tels que des particules de peau, de graisse et/ou de perspiration, un dispositif d'analyse des minéraux (32) pour déterminer la teneur en minéraux des poils corporels (10) et un détecteur de pH (34) pour déterminer le pH des substances corporelles (18, 20, 22) retirées avec ceux-ci et/ou un dispositif de mesure de la conductance (36), **caractérisé en ce que** le dispositif soit intégré dans une station de nettoyage (6, 6', 6") pour un rasoir (4) ou que le dispositif soit intégré dans un rasoir, particulièrement un rasoir à sec ou électrique (4).

9. Dispositif selon la revendication 8 avec un dispositif pour la mesure de la longueur des poils (24), en particulier un dispositif de mesure de la longueur des poils optique, gravimétrique ou de criblage (24).

10. Dispositif selon la revendication 8 ou 9, dans lequel le dispositif de séparation (14, 14") a un dispositif de filtration et/ou un dispositif de séparation à contre-courant et/ou un dispositif de séparation par ultrason.

11. Dispositif selon l'une quelconque des revendications 8 à 10, dans lequel un appareil d'incinération par micro-onde est organisé sur le dispositif d'analyse des minéraux (26).

12. Dispositif selon n'importe lesquelles des revendications 8 à 11, dans lequel le dispositif d'analyse des minéraux (26) a un spectroscope, en particulier un spectromètre à émission avec un plasma couplé de manière inductive, en particulier du plasma d'argon, ou un spectromètre UV-VIS.

13. Utilisation d'un système de rasage (2, 2 ', 2 ") comme défini dans fune quelconque des revendications 8 à 12, comprenant un rasoir à sec ou électrique, pour la détermination des paramètres du corps de son utilisateur.
